# EUROPEAN PATENT APPLICATION

(11) **EP 1 710 318 A1**
(43) Date of publication of application: **11.10.2006**
(21) Application number: 04806991.8
(22) Date of filing: 14.12.2004
(51) Int. Cl.: C12Q 1/04

(54) **METHOD OF CONVENIENTLY DETECTING STRAIN PRODUCING CLASS C BETA-LACTAMASE**

(30) Priority: 15.12.2003 JP 2003416165
(71) Applicant: Japan Health Sciences Foundation, Tokyo 103-0001 (JP)
(72) Inventor: YAGI, Tetsuya; c/o National Institute of, Musashimurayama-shi, Tokyo; 2080011 (JP); WACHINO, Jun-ichi; c/o National Institute of, Musashimurayama-shi, Tokyo; 2080011 (JP); ARAKAWA, Yoshichika; c/o National Institute of, Musashimurayama-shi, Tokyo; 2080011 (JP)
(74) Representative: Zimmermann, Gerd Heinrich
(86) International application number: PCT/JP2004/018630
(87) International publication number: WO 2005/056820

(57) **Abstract**

A method for determining whether a test bacterium is a class C beta-lactamase-producing bacterium. Spots of a class C beta-lactamase inhibitor and a beta-lactam drug are applied at an interval on the surface of a solid medium that has been coated with the test bacterium, the solid medium is cultured, and following culturing, determining whether or not the inhibitory zone formed around the beta-lactam drug has extended toward the class C beta-lactamase inhibitor. A mixture of class C beta-lactamase inhibitor and beta-lactam drug and a beta-lactam drug in spots are applied at an interval on the surface of a solid medium that has been coated with the test bacterium, the solid medium is cultured, and following culturing, observing the difference between the inhibitory zone formed around the mixture and the inhibitory zone formed around the beta-lactam drug. A kit for determining class C beta-lactamase-producing bacteria, in which a disk containing a class C beta-lactamase inhibitor and a disk containing a beta-lactam drug are arranged on a striplike base. A kit for determining class C beta-lactamase-producing bacteria, in which a disk containing both a class C beta-lactamase inhibitor and a beta-lactam drug and a disk containing a beta-lactam drug are arranged on a striplike base. Provided are a method for readily detecting class C beta-lactamas-producing bacterium and kits performing the method.

## Description

### [Technical Field]

The present invention relates to a method for readily detecting class C beta-lactamase-producing bacteria employing an inhibitor highly specific to class C beta-lactamases.

### [Background Technology]

Beta-Lactamase production is an important tolerance mechanism for beta-lactams that is employed by gram-negative bacteria including the family Enterobacteriaceae. That is, beta-lactamases are bacterial enzymes that hydrolyzes the beta-lactam ring of betalactamantibiotics, deteriorating their antibacterial activity.

### [Types of beta-lactamase and research to-date on methods of detecting bacteria producing the same]

Beta-Lactamases come in classes A, B, C, and D based on structural characteristics. The present inventors developed the Twin test (a modified disk diffusion test) as a method of detecting class A extended-spectrum beta-lactamases (ESBLs), which has been a major problem clinically, and the SMA method as a method of detecting class B metallo-beta-lactamases (Japanese Unexamined Patent Publication (KOKAI) No. 2000-224998)), and have seen them enter into wide use.

Genetic determinants for class C beta-lactamases are mainly present in the chromosomes of numerous bacterial species that cause infection in hospitals, such as *Pseudomonas aeruginosa, Enterobacter spp., Citrobacter freundii,* and *Escherichia coli.* Although not yet reported in great number, cases have appeared in Japan where this class of beta-lactamases have been mediated by plasmids. Class C beta-lactamases impart resistance to penicillins and cephalosporins and is thus clinically problematic. Hence, there is a need to develop of a method of readily detecting bacteria that produce class C beta-lactamases.

A method showing the derivation of class C beta-lactamase-producing bacteria with cefoxitin, a three-dimensional method employing a crude enzyme solution, and the like, have been reported as methods of detecting class C beta-lactamases. However, these methods all possesse difficulties in terms of simplicity of determination, ease of use, and clarity of result interpretation, thus they are not widely employed to date.

Accordingly, the object of the present invention is to provide a method for readily detecting class C beta-lactamases. More particularly, the object of the present invention lies in providing a feasible method of detecting class C beta-lactamase-producing bacteria that is simple enough for routine use in the microbiology laboratories of hospitals.

Further, the present invention provides a kit for more simply implementing the determination of class C beta-lactamase-producing bacteria and a method for determining class C beta-lactamase-producing bacteria using this kit.

### [Disclosure of the Invention]

The present invention, which solves the above-stated problems, comprises the following:
[1] A method for determining whether a test bacterium is a class C beta-lactamase-producing bacterium by applying spots of a class C beta-lactamase inhibitor and a beta-lactam drug, respectively, at an interval on the surface of a solid medium that has been coated with the test bacterium, culturing the solid medium, and following culturing, determining whether or not the inhibitory zone formed around the beta-lactam drug has extended toward the class C beta-lactamase inhibitor.
[2] The method according to [1], wherein the interval between the class C beta-lactamase inhibitor and the beta-lactam drug is set so that the range of diffusion of the class C beta-lactamase inhibitor and the range of diffusion of the beta-lactam drug overlap during the culture period.
[3] The method according to [1] or [2], wherein a disk containing class C beta-lactamase inhibitor and a disk containing a beta-lactam drug are employed to apply the class C beta-lactamase inhibitor and beta-lactam drug in spots.
[4] A method for determining whether a test bacterium is a class C beta-lactamase-producing bacterium by applying a mixture of class C beta-lactamase inhibitor and beta-lactam drug and a beta-lactam drug in spots at an interval on the surface of a solid medium that has been coated with the test bacterium, culturing the solid medium, and following culturing, observing the difference between the inhibitory zone formed around the mixture and the inhibitory zone formed around the beta-lactam drug.
[5] The method according to [4], wherein a disk containing both class C beta-lactamase inhibitor and the beta-lactam drug and a disk containing the beta-lactam drug are employed to apply the mixture of class C beta-lactamase inhibitor and beta-lactam drug and the beta-lactam drug in spots.
[6] The method according to any of [1] to [5], wherein the class C beta-lactamase inhibitor is a boronic acid compound.
[7] The method according to [6], wherein the boronic acid compound is 3-aminophenylboronic acid.
[8] The method according to any of [1] to [7], wherein the beta-lactam drug is a third generation cephalosporin.
[9] The method according to [8], wherein the third generation cephalosporin is ceftazidime or cefotaxime.
[10] A kit for determining class C beta-lactamase-producing bacteria, characterized in that a disk containing a class C beta-lactamase inhibitor and a disk containing a beta-lactam drug are arranged on a striplike base.
[11] A kit for determining class C beta-lactamase-producing bacteria, characterized in that a disk containing both a class C beta-lactamase inhibitor and a beta-lactam drug and a disk containing a beta-lactam drug are arranged on a striplike base.
[12] A method for determining whether a test bacterium is a class C beta-lactamase-producing bacterium by placing the kit according to [10] or [11] on the surface of a solid medium that has been coated with the test bacterium, culturing, and following culturing, observing differences in the inhibitory zones formed around the two disks.
[13] A method for determining whether a test bacterium is a class C beta-lactamase-producing bacterium by preparing multiple liquid media containing stepwise diluted concentrations of beta-lactam drug and equal concentrations of a class C beta-lactamase inhibitor, inoculating the test bacterium into each of the liquid media, culturing, and following culturing, observing the decrease in MIC.
[14] The method according to [13], wherein the test bacterium is determined to be a class C beta-lactamase-producing bacterium when the decrease in MIC is eightfold or greater.
[15] The method according to [13] or [14], wherein the class C beta-lactamase inhibitor is a boronic acid compound.
[16] The method according to [15], wherein the boronic acid compound is 3-aminophenylboronic acid.
[17] The method according to any of [13] to [16], wherein the beta-lactam drug is a third generation cephalosporin.
[18] The method according to [17], wherein the third generation cephalosporin is ceftazidime or cefotaxime.

According to the present invention, class C beta-lactamase-producing bacteria can be determined by a method simple enough for routine use in hospital microbiology laboratories.
The present invention further provides a kit for implementing a method for more readily determining class C beta-lactamase-producing bacteria and a method employing this kit to determine class C beta-lactamase-producing bacteria.

### [Best Mode of Implementing the Invention]

As described further below, boronic acid compounds, a monobactam derivative Syn2190, and the like, have been reported to inhibit class C beta-lactamase. However, no method of determining class C beta-lactamase-producing bacteria using these inhibitors is known.

### [First Aspect of the Method of the Present Invention]

The first aspect of the method of the present invention is a method for determining whether a test bacterium is a class C beta-lactamase-producing bacterium by applying spots of class C beta-lactamase inhibitor and a beta-lactam drug at an interval on the surface of a solid medium that has been coated with the test bacterium, culturing the solid medium, and following culturing, determining whether or not the inhibitory zone formed around the beta-lactam drug has extended toward the class C beta-lactamase inhibitor.

The solid medium employed in the present invention can be any of the usual solid media widely employed in a drug susceptibility test and the like. For example, the solid medium can be an agar medium containing nutrients such as a carbon source and a nitrogen source. An example of such a solid medium is Mueller-Hinton agar medium (Difco).

The test bacterium is coated on the surface of the above-described solid medium. The method and conditions under which the bacterium is applied to the surface of the solid medium may be identical to those commonly employed in drug susceptibility test. For example, the Standard Methods of the Japan Chemotherapy Society or the disk diffusion methods established by, the National Committee for Clinical Laboratory Standards (NCCLS) (now the Clinical Laboratory Standards Institute (CLSI)), may be employed to apply the bacterium on Mueller-Hinton agar medium.

The class C beta-lactamase inhibitor and beta-lactam drug are then spotted with an interval between them on the surface of the solid medium on which the test bacterium has been coated. When applying the spots, a disk containing a beta-lactam drug and a disk containing a class C beta-lactamase inhibitor may be suitably employed. Commercially available disks containing beta-lactam drugs may be employed. The beta-lactam drug may be suitably selected from among those commercially available as treatment drugs. Examples of the beta-lactam drug are third generation cephalosporins and cephamycins. Examples of third generation cephalosporins and cephamycins are ceftazidime, latamoxef, cefmenoxime, and cefotaxime. However, no limitation to these drugs is intended. A disk that is not commercially available can also be prepared by impregnating filter paper of suitable size and shape with a beta-lactam drug, employing a solvent as needed.

A disk containing a class C beta-lactamase inhibitor can be prepared by impregnating filter paper of suitable size and shape with a class C beta-lactamase inhibitor. The class C beta-lactamase inhibitor can be suitably selected from among drugs having an inhibiting effect on class C beta-lactamase. Examples the drugs are boronic acid compounds, boric acid, monobactam derivatives, and phenylacetylglycyl heterocycle derivatives. Examples of boronic acid compounds are 3-aminophenylboronic acid, 3-nitrophenylboronic acid, and 2-thiophenylboronic acid, and benzo[b]thiophene-2-boronic acid.

The quantities employed in the spots of beta-lactam drug and class C beta-lactamase-inhibitor are suitably determined taking into account the diffusion properties of the individual drug on the surface of the solid medium, the culture (diffusion) period, and the strength of the inhibiting effect on class C beta-lactamase. For example, for beta-lactam drugs, a quantity of 30 micrograms is suitably employed for ceftazidime. For class C beta-lactamase inhibitors, a quantity falling within a range of 300 to 500 micrograms is suitable for 3-aminophenylboronic acid. However, these are only yardsticks, and it is possible to suitably vary these quantities based on the type of test bacterium, shape and size of the inhibitory zone formed around the beta-lactam drug, and the like.

The interval between the class C beta-lactamase inhibitor and the beta-lactam drug is suitably set so that the range of diffusion of the class C beta-lactamase inhibitor and the range of diffusion of the beta-lactam drug overlap during the culture period from the perspective of detecting a class C beta-lactamase-producing bacterium by utilizing the interaction between the two.

The ranges of diffusion of the beta-lactam drug and the class C beta-lactamase inhibitor vary with the type of drug, quantities employed in the spots, and culture conditions (principally time). Thus, a center-to-center interval between the class C beta-lactamase inhibitor and beta-lactam drug of about 1 to 2 cm, for example, is suitably employed so that the diffusion ranges of the two overlap.

The solid medium on the surface of which the class C beta-lactamase inhibitor and beta-lactam drug have been positioned is cultured. For example, culture conditions ranging from 35 to 37°C and a period of 12 to 36 hours are possible. However, the culture conditions, particularly time, are suitably determined by taking into account the drug diffusion ranges.

The above-described culturing causes the beta-lactam drug and class C beta-lactamase inhibitor that have been positioned on the surface of the solid medium to diffuse across the surface and into the solid medium. When the test bacterium is a class C beta-lactamase-producing bacterium, it produces class C beta-lactamase and becomes less sensitive to the beta-lactam drug. Accordingly, since only a beta-lactam drug has been placed on the surface of the solid medium, either no inhibitory zone is observed or only a small inhibitory zone forms in the vicinity of the disk.

When a class C beta-lactamase inhibitor is positioned at a certain interval from a beta-lactam drug, a bacterial growth inhibitory zone will be observed around the beta-lactam drug positioned so that the diffusion ranges of the beta-lactam drug and class C beta-lactamase inhibitor overlap even when the test bacterium is a class C beta-lactamase-producing bacterium. This is because the class C beta-lactamase inhibitor inhibits the activity of the class C beta-lactamase, causing growth of the bacterium to be inhibited by the beta-lactam drug. Here, the shape of the inhibition zone that is observed will depend on the extent of overlap of the diffusion range of the beta-lactam drug and the diffusion range of the class C beta-lactamase inhibitor, but will generally be distorted. That is, the inhibitory zone formed around the beta-lactam drug will expand toward the class C beta-lactamase inhibitor. The inhibitory zone with a varied shape can be clearly distinguished based on size from an inhibitory zone formed around a beta-lactam drug at a position not overlapping with the diffusion range of the class C beta-lactamase inhibitor.

There are two cases where a test bacterium is not a class C beta-lactamase-producing bacterium. There is the case of a sensitive bacteria, where the growth of the bacterium is impeded by the beta-lactam drug and a large inhibitory zone is formed, and the case of a bacterium that does not produce class C beta-lactamase, where the beta-lactam drug does not impede the growth of the bacterium and no inhibitory zone is formed (for example, the case of a class A or B beta-lactamase-producing bacterium). In the former, since the bacterium does not produce class C beta-lactamase, detection is possible by means of a drug sensitivity test employing only the beta-lactam drug. That is, even when not employing a class C beta-lactamase inhibitor, a large, undistorted inhibitory zone will form around the beta-lactam drug. In the latter, in a drug sensitivity test employing only a beta-lactam drug without a class C beta-lactamase inhibitor, it is impossible to determine whether the bacterium produces class C beta-lactamase. By contrast, in the method of the present invention, when a class C beta-lactamase inhibitor is positioned at a close interval, either no inhibitory zone forms around the beta-lactam drug, or an inhibitory zone forms but the class C beta-lactamase inhibitor does not cause any distortion or deformation to the zone. Accordingly, the method of the present invention permits distinction between bacteria that produce class C beta-lactamase and bacteria that produce class A or B beta-lactamase.

### [Second Aspect of the Method of the Present Invention]

The second aspect of the method of the present invention is a method for determining whether a test bacterium is a class C beta-lactamase-producing bacterium by applying a mixture of class C beta-lactamase inhibitor and beta-lactam drug and a beta-lactam drug in spots at an interval on the surface of a solid medium that has been coated with the test bacterium, culturing the solid medium, and following culturing, observing the difference between the inhibitory zone formed around the mixture and the inhibitory zone formed around the beta-lactam drug.

The solid medium, class C beta-lactamase inhibitor, and beta-lactam drug employed in the second aspect of the present invention are identical to those employed in the first aspect of the method of the present invention. The solid medium culturing conditions are also identical to those employed in the first aspect of the method of the present invention.

In the second aspect of the method of the present invention, in the course of applying spots of a mixture of class C beta-lactamase inhibitor and beta-lactam drug and a beta-lactam drug, a disk containing class C beta-lactamase inhibitor and beta-lactam drug and a disk containing beta-lactam drug are desirably employed. The disk containing the beta-lactam drug is identical to that employed in the first aspect of the method of the present invention. The disk containing class C beta-lactamase inhibitor and beta-lactam drug can be prepared by further impregnating a disk containing beta-lactam drug with class C beta-lactamase inhibitor.

In the second aspect of the method of the present invention, in contrast to the first aspect of the method of the present invention, it is not necessary that the interval between the mixture of class C beta-lactamase inhibitor and beta-lactam drug and the beta-lactam drug be set so that the range of diffusion of the class C beta-lactamase inhibitor in the mixture overlaps the range of diffusion of the beta-lactam drug (not in the mixture) during culturing. It is set so that the ranges of diffusion of the drug from individual spots (preferably each disk) do not overlap. For example, the spots are formed 3 cm or more apart based on their center-to-center interval.

In the case of the beta-lactam drug alone, the action of class C beta-lactamase produced by a class C beta-lactamase-producing microbe results in growth of the bacterium being inhibited little or not at all. Accordingly, no or only a small inhibitory zone is observed.

By contrast, in the case of the mixture of beta-lactam drug and class C beta-lactamase inhibitor, even when the bacterium produces class C beta-lactamase, a large inhibitory zone will be observed. This is because the class C beta-lactamase inhibitor impedes the activity of the class C beta-lactamase, and as a result, the beta-lactam drug hinders growth of the bacterium. Accordingly, this can be clearly distinguished by the size of the inhibitory zone (although not always formed) forming around just the beta-lactam drug. That is, if an expansion of 5 mm or more is observed in the diameter of the inhibitory zone, a determination can be made that the bacterium produces class C beta-lactamase.

Additionally, there are two cases where the test bacterium does not produce class C beta-lactamase. There is the case of a sensitive bacterium where the beta-lactam drug impedes growth of the bacterium and a large inhibitory zone forms, and the case where the bacterium does not produce class C beta-lactamase, but growth of the bacteria is not impeded by the beta-lactam drug and no inhibitory zone forms (for example, the case where the bacterium produces class A or B beta-lactamase). In the former case, since the bacterium does not produce class C beta-lactamase, it can be determined by a drug sensitivity test employing just a beta-lactam drug. That is, determination is possible in that inhibitory zones of roughly equal size form around both disks regardless of whether or not they contain a class C beta-lactamase inhibitor. In the latter case, it is impossible to determine whether or not the bacterium produces class C beta-lactamase by a drug sensitivity test employing just a beta-lactam drug without a class C beta-lactamase inhibitor. By contrast, in the method of the present invention, regardless of whether a class C beta-lactamase inhibitor is contained, no inhibitory zones form, or only small inhibitory zones form, and the size of the two inhibitory zones is roughly identical. Accordingly, the method of the present invention permits distinction between class C beta-lactamase-producing bacteria and class A and B beta-lactamase-producing bacteria.

### [The kit of the present invention]

The kit for determining class C beta-lactamase-producing bacteria of the present invention comes in the form of:
(1) A kit for determining class C beta-lactamase-producing bacteria, characterized in that a disk containing a class C beta-lactamase inhibitor and a disk containing a beta-lactam drug are arranged on a striplike base; and (2) a kit for determining class C beta-lactamase-producing bacteria, characterized in that a disk containing both a class C beta-lactamase inhibitor and a beta-lactam drug and a disk containing a beta-lactam drug are arranged on a striplike base.
   Kit (1) for determining class C beta-lactamase-producing bacteria is employed in the first aspect of the method of the present invention as set forth above, and kit (2) for determining class C beta-lactamase-producing bacteria is employed in the second aspect of the method of the present invention as set forth above.

The same disk containing a beta-lactam drug as employed in the above-described methods of the present invention may be employed in the kits of the present invention. Further, the disk containing a class C beta-lactamase inhibitor may be in the form of a disk of filter paper or the like that is impregnated with a class C beta-lactamase inhibitor. Still further, the disk containing both class C beta-lactamase inhibitor and beta-lactam drug may be in the form of a disk containing a beta-lactam drug that is further impregnated with a class C beta-lactamase inhibitor. These disks are arranged in a row on a striplike base.

Neither the shape nor the dimensions of the striplike base are specifically limited. They can be suitably determined by considering the size of the solid medium with which the kit is employed. To facilitate reading of the inhibitory zones, the striplike base can be made of a highly transparent material. The spacing of the individual disks, the content of beta-lactam drug on the disk, and the like, can be suitably determined by taking into account the same points as set forth above in the method of the present invention.

In the method for determining class C beta-lactamase-producing bacteria employing the above-described kit of the present invention, the kit is placed on the surface of a solid medium that has been coated with the test bacterium, culturing is conducted, and following culturing, a determination is made as to whether or not the test bacterium is a class C beta-lactamase-producing bacterium based on the difference between the inhibitory zones formed around the two disks. In this method, with the exception that the above-described kit is employed, the above-described method of the present invention may be employed as is.

### [Third aspect of the method of the present invention]

The third aspect of the method of the present invention is a method based on a trace quantity liquid dilution method. In this method, multiple liquid media containing stepwise diluted concentrations of beta-lactam drug and equal concentrations of a class C beta-lactamase inhibitor are prepared. For example, Mueller-Hinton liquid medium (Difco) may be employed as the liquid medium.
The beta-lactam drug and class C beta-lactamase inhibitor are identical to those described in the first aspect of the method of the present invention.
For example, the degree of stepwise dilution of the beta-lactam drug in the liquid medium falls within a range of from 0.5 to 256 micrograms/mL.
Further, the concentration of the class C beta-lactamase inhibitor in the individual liquid media is identical. For example, in the case of 3-aminophenylboronic acid, it can be 200 micrograms/mL.

The test bacterium is inoculated into each of the liquid media thus prepared and cultured. The same culture conditions may be employed as when employing a solid medium.

Following culturing, a determination can be made as to whether or not the test bacterium is a class C beta-lactamase-producing bacterium based on the decrease in the MIC. Specifically, when the decrease in MIC is eightfold or greater, the test bacterium can be determined to be a class C beta-lactamase-producing bacterium.

### [Embodiments]

The present invention is described in greater detail below through embodiments.

### Embodiment 1 (First aspect of the method of the present invention)

Test bacteria were inoculated onto a Mueller-Hinton agar medium by a method based on the disk diffusion method recommended by the NCCLS. The test bacteria employed are given in Table 1 below.

**[Table 1]**

| Designation of test bacteria |
|---|
| *E. cloacae* HKY226 |
| *C. freundi* HKY543 |
| *S. marcescens* S94 |
| *P. aeruginosa* 03-192 |
| *K. pneumoniae* MOX-1 |
| *E. coli* CMY-2 |
| *K. pneumoniae* DHA-1 [+CTX-M9] |
| *K. pneumoniae* SHV-12 Class A |
| *K. pneumoniae* IMP-1 Class B |

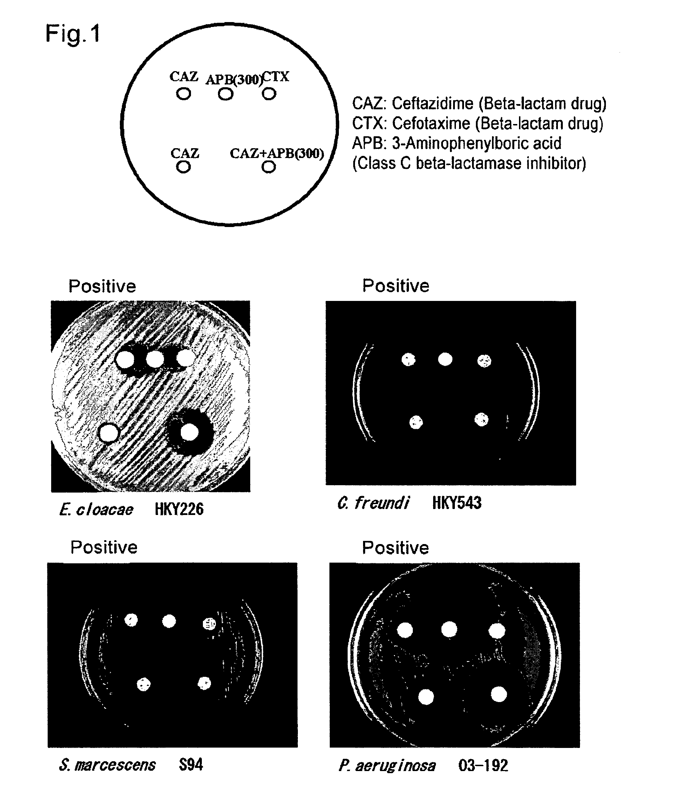

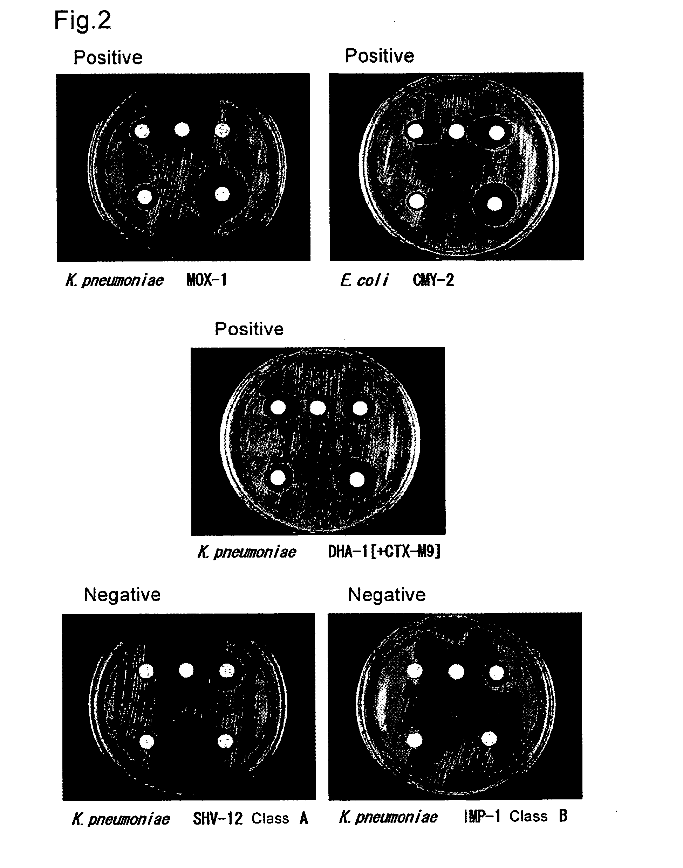

A disk (made by Eiken Kagaku, 6 mm in diameter) impregnated from the top with 300 micrograms of APB was placed onto the medium. KB disks (6 mm in diameter) of ceftazidime (CAZ) and cefotaxime (CTX) were positioned to the left and right of the disk at a center-to-center interval of about 18 mm. From the left, the three disks in the top section of the Petri dish shown in Figs. 1 and 2 are CAZ, APB, and CTX. Table 2 gives the status of growth-inhibitory band and shape of growth.

**[Table 2]**

| Designation of test bacterium | |
|---|---|
| *E. cloacae* HKY226 | When the disks were positioned 10 mm apart center-on-center, inhibitory zones looking as if they were being drawn toward the APB disk were observed around CAZ and CTX. Positive. |
| *C. freundi* HKY543 | No inhibitory zone was observed around the APB disk, but inhibitory zones were observed around the CAZ and CTX disks. Both were deformed as if drawn toward the APB disk. Positive. |
| *S. marcescens* S94 | Inhibitory zones were observed around both the CAZ and CTX disks, both looking as if drawn toward the APB disk. Positive. |
| *P. aeruginosa* 03-192 | Inhibitory zone only observed around the CAZ disk, expanding in a deformed manner toward the APB disk. Positive. |
| K. pneumoniae MOX-1 | Identical to *P. aeruginosa* 03-192 (Positive). |
| *E. coli* CMY-2 | Identical to *S. marcescens* S94 (Positive). |
| *K. pneumoniae* DHA-1 [+CTX-M9] | Inhibitory zones were observed around both the CAZ and CTX disks. The CAZ ring was deformed as if drawn toward the APB disk. Positive. |
| *K. pneumoniae* SHV-12 Class A | An inhibitory zone was observed around the CTX disk, but did not appear deformed in the direction of the APB disk. Negative. |
| *K. pneumoniae* IMP-1 | Identical to SHV-12 (Negative). |
| Class B | |

As shown in Figs. 1 and 2, the growth inhibitory bands around CAZ and CTX following overnight culturing looked deformed as if drawn toward the APB disk in the case of class C beta-lactamase-producing bacteria. That is, the inhibitory zones forming around the beta-lactam drug (CAZ or CTX) expanded toward the class C beta-lactamase inhibitor (APB), and the test bacterium could be determined to be a class C beta-lactamase-producing bacterium. By contrast, no distortion in growth-inhibitory zones was observed for class A and B beta-lactamase-producing bacteria that did not produce class C beta-lactamase.

### Embodiment 2 (Second aspect of the method of the present invention)

In the same manner as in Embodiment 1, the test bacterium was inoculated onto a Mueller-Hinton agar medium, and KB disks (6 mm in diameter) of CAZ were positioned at least 3 cm apart center-on-center. One of the CAZ disks was impregnated with 300 micrograms of APB. After overnight culturing, the diameters of the growth inhibitory zones around the two CAZ disks were measured. From the left, the two disks in the lower portions of the Petri dishes shown in Figs. 1 and 2 are CAZ and CAZ+APB disks. The results are given with comments in Table 3 below.

**[Table 3]**

| Designation of test bacterium | |
|---|---|
| *E. cloacae* HKY226 | No inhibitory zone was observed around the CAZ disk. An inhibitory zone was observed around the CAZ+APB disk, and the expansion of the inhibition disk diameter was at least 5 mm. Positive. |
| *C. freundi* HKY543 | A small inhibitory zone was observed around the CAZ disk. An enlargement of at least 5 mm was observed in the diameter of the inhibitory zone around the CAZ+APB disk. Positive. |
| *S. marcescens* S94 | Identical to *C. freundii* HKY543. |
| *P. aeruginosa* 03-192 | Identical to *C. freundii* HKY543. |
| *K. pneumoniae* | Identical to *C. freundii* HKY543. |
| MOX-1 | |
| *E. coli* CMY-2 | Identical to *C. freundii* HKY543. |
| *K. pneumoniae* DHA-1 [+CTX-M9] | Identical to *C. freundii* HKY543. |
| *K. pneumoniae* SHV-12 Class A | No inhibitory zones observed around CAZ or CAZ+APB disks. Negative. |
| *K. pneumoniae* IMP-1 Class B | Identical to above (SHV-12). |

As shown in Figs. 1 and 2, class C beta-lactamase-producing bacteria exhibited enlarged diameters of 5 mm or more (relative to disks not impregnated with APB) and could be determined to be class C beta-lactamase-producing bacteria. By contrast, class A and B beta-lactamase-producing bacteria that did not produce class C beta-lactamase did not exhibit enlarged growth inhibitory zones.

### Embodiment 3 (Third aspect of the method of the present invention)

Mueller-Hinton liquid media with stepwise diluted concentrations of CAZ and Mueller-Hinton liquid media to which 200 micrograms of APB was added to the stepwise diluted concentration of CAZ were prepared and inoculated by a method based on the trace quantity liquid dilution method recommended by the NCCLS. Following overnight culturing, the MIC was measured. The results are given in Table 4.

**[Table 4]**

| Bacterial strain (producing beta-lactamase) | MIC (micrograms/mL) | | | |
|---|---|---|---|---|
| | CAZ | CAZ+CA | CAZ+SMA | CAZ+APB |
| *Klebsiella pneumoniae* | | | | |
| HKY402 (SHV-12: Class A) | >512 | ≤0.5 | 512 | >512 |
| KP 115 (IMP-1: Class B) | 256 | 256 | ≤0.5 | 512 |
| NU2936 (MOX-1: Class C) | 64 | 64 | 32 | 1 |
| *E. cloacae* HKY226 | 256 | - | - | 32 |
| *C. freundii* HKY543 | 64 | - | - | 1 |
| *S. marcescens* S94 | 64 | - | - | 2 |
| *P. aeruginosa* NCB03-192 | 16 | - | - | 2 |
| *K. pneumoniae* NCB02189 DHA-1+CTX-M9 | 64 | - | - | 8 |
| *E. coli* NS12 CMY-2 | 64 | - | - | 4 |

| | | | | |
|---|---|---|---|---|
| CAZ: ceftazidime; CA: clavulanic acid; SMA: sodium mercaptoacetate; APB: 3-aminophenylboronic acid CA was employed in a concentration of 4 micrograms/mL, SMA in a concentration of 500 micrograms/ml, and APB in a concentration of 200 micrograms/mL. | | | | |

When the decrease in MIC of CAZ+APB was eightfold or more relative to the MIC of CAZ alone, the bacterium was determined to produce class C beta-lactamase. The bacteria determined to be class C beta-lactamase-producing bacteria in Embodiments 1 and 2 all exhibited a drop in MIC of eightfold or more. By contrast, class A and B beta-lactamase-producing bacteria that did not produce class C beta-lactamase did not exhibit a drop in MIC.
CAZ+CA exhibited a drop in MIC of eightfold or more relative to CAZ alone for class A and B beta-lactamase-producing bacteria, and CAZ+SMA exhibited a drop in MIC of eightfold or more relative to CAZ alone only for class B beta-lactamase-producing bacteria.

### [Industrial Applicability]

The present invention provides a simple method for detecting class C beta-lactamase-producing bacteria that is easy enough for use even in a hospital examination room.

### [Brief Description of the Figures]

Fig. 1 shows the results of Embodiments 1 and 2.
Fig. 2 shows the results of Embodiments 1 and 2.

## Claims

1. A method for determining whether a test bacterium is a class C beta-lactamase-producing bacterium by applying spots of a class C beta-lactamase inhibitor and a beta-lactam drug at an interval on the surface of a solid medium that has been coated with the test bacterium, culturing the solid medium, and following culturing, determining whether or not the inhibitory zone formed around the beta-lactam drug has extended toward the class C beta-lactamase inhibitor.

2. The method according to claim 1, wherein the interval between the class C beta-lactamase inhibitor and the beta-lactam drug is set so that the range of diffusion of the class C beta-lactamase inhibitor and the range of diffusion of the beta-lactam drug overlap during the culture period.

3. The method according to claim 1 or 2, wherein a disk containing class C beta-lactamase inhibitor and a disk containing a beta-lactam drug are employed to apply the class C beta-lactamase inhibitor and beta-lactam drug in spots.

4. A method for determining whether a test bacterium is a class C beta-lactamase-producing bacterium by applying a mixture of class C beta-lactamase inhibitor and beta-lactam drug and a beta-lactam drug in spots at an interval on the surface of a solid medium that has been coated with the test bacterium, culturing the solid medium, and following culturing, observing the difference between the inhibitory zone formed around the mixture and the inhibitory zone formed around the beta-lactam drug.

5. The method according to claim 4, wherein a disk containing the class C beta-lactamase inhibitor and the beta-lactam drug and a disk containing the beta-lactam drug are employed to apply the mixture of class C beta-lactamase inhibitor and beta-lactam drug and the beta-lactam drug in spots.

6. The method according to any of claims 1 to 5, wherein the class C beta-lactamase inhibitor is a boronic acid compound.

7. The method according to claim 6, wherein the boronic acid compound is 3-aminophenylboronic acid.

8. The method according to any of claims 1 to 7, wherein the beta-lactam drug is a third generation cephalosporin.

9. The method according to [8], wherein the third generation cephalosporin is ceftazidime or cefotaxime.

10. A kit for determining class C beta-lactamase-producing bacteria, **characterized in that** a disk containing a class C beta-lactamase inhibitor and a disk containing a beta-lactam drug are arranged on a striplike base.

11. A kit for determining class C beta-lactamase-producing bacteria, **characterized in that** a disk containing both a class C beta-lactamase inhibitor and a beta-lactam drug and a disk containing a beta-lactam drug are arranged on a striplike base.

12. A method for determining whether a test bacterium is a class C beta-lactamase-producing bacterium by placing the kit according to claim 10 or 11 on the surface of a solid medium that has been coated with the test bacterium, culturing, and following culturing, observing differences in the inhibitory zones formed around the two disks.

13. A method for determining whether a test bacterium is a class C beta-lactamase-producing bacterium by preparing multiple liquid media containing stepwise diluted concentrations of beta-lactam drug and equal concentrations of a class C beta-lactamase inhibitor, inoculating the test bacterium into each of the liquid media, culturing, and following culturing, observing the decrease in MIC.

14. The method according to claim 13, wherein the test bacterium is determined to be a class C beta-lactamase-producing bacterium when the decrease in MIC is eightfold or greater.

15. The method according to claim 13 or 14, wherein the class C beta-lactamase inhibitor is a boronic acid compound.

16. The method according to claim 15, wherein the boronic acid compound is 3-aminophenylboronic acid.

17. The method according to any of claims 13 to 16, wherein the beta-lactam drug is a third generation cephalosporin.

18. The method according to claim 17, wherein the third generation cephalosporin is ceftazidime or cefotaxime.
